## Europäisches Patentamt

## European Patent Office

## Office européen des brevets

(11) Veröffentlichungsnummer: **0 077 510**
**B1**

(12) **EUROPÄISCHE PATENTSCHRIFT**

(45) Veröffentlichungstag der Patentschrift:
06.04.88

(21) Anmeldenummer: 82109363.0

(22) Anmeldetag: 09.10.82

(51) Int. Cl.⁴: **A 61 L 15/00,** A 01 N 25/04,
A 01 N 25/10, A 24 D 3/00,
A 61 K 9/22

(54) Absorptionsmaterial für Wasser, wässrige Lösungen und wässrige Körperflüssigkeiten.

(30) Priorität: 16.10.81 DE 3141098

(43) Veröffentlichungstag der Anmeldung:
27.04.83 Patentblatt 83/17

(45) Bekanntmachung des Hinweises auf die Patenterteilung:
06.04.88 Patentblatt 88/14

(84) Benannte Vertragsstaaten:
BE CH DE FR GB IT LI SE

(56) Entgegenhaltungen:
EP-A-0 002 603
FR-A-2 006 324
FR-A-2 080 724
FR-A-2 122 432
FR-A-2 173 934
FR-A-2 319 434
FR-A-2 402 474

Die Akte enthält technische Angaben, die nach
dem Eingang der Anmeldung eingereicht wurden
und die nicht in dieser Patentschrift enthalten sind.

(73) Patentinhaber: Chemische Fabrik Stockhausen
GmbH, Bäkerpfad 25, D-4150 Krefeld (DE)

(72) Erfinder: Chmelir, Miroslav, Dr., Grönkesdyk 36,
D-4150 Krefeld (DE)
Erfinder: Künschner, Alois, Dr., Kimplerstrasse 96,
D-4150 Krefeld (DE)

(74) Vertreter: Klöpsch, Gerald, Dr.- Ing., An Gross St.
Martin 6, D-5000 Köln 1 (DE)

EP 0 077 510 B1

**Beschreibung**

Die Erfindung betrifft ein Absorptionsmaterial, Verfahren zu seiner Herstellung sowie seine Verwendung als Absorptionsmittel für Wasser, Wasserdampf, wässrige Lösungen, wässrige oder seröse Körperflüssigkeiten wie Urin oder Blut sowie zur Aufnahme, Zurückhaltung und späteren gesteuerten Abgabe von Wasser, wässrigen Lösungen und/oder den darin gelösten Komponenten an andere Körper.

In den letzten Jahren wurde eine Anzahl verschiedener Polymerisate entwickelt, die hohes Absorptionsvermögen für Wasser und Körperflüssigkeiten aufweisen. Die meisten Produkte wurden auf Stärkebasis, wie z.B. Stärke-Acrylnitril-Pfropfpolymerisate (US-PS 3 997 484, 3 661 815, 4 155 888, 3 935 099), gelatinisierte Stärkederivate (DE-OS 2 702 781), Stärke-Acrylamid-Acrylamidopropansulfonsäure-Pfropfpolymerisat oder auf Cellulosebasis, wie Derivate von Alkyl- oder Hydroxyalkylcellulose (JA-PS 11/125.481), Carboxymethylcellulose (BE-PS 862 130, GB-PS 1 159 949) und auf Polysaccharidbasis (DE-OS 26 50 377) hergestellt. Zu den vollsynthetischen in zahlreichen Patenten beschriebenen Absorptionsmitteln gehören vernetzte Polymere und Copolymere auf Acryl- oder Methacrylsäurebasis (DE-OS 24 29 236, DE-OS 26 14 662, US-PS 4,018,951, US-PS 3,926,891, US-PS 4,066,583, US-PS 4,062,817, DE-OS 27 12 043, DE-OS 26 53 135, DE-OS 28 13 634) oder Maleinsäure-Derivate (US-PS 4,041,228).

Alle diese Produkte sind praktisch wasserunlöslich und absorbieren das Vielfache ihres Gewichts an Wasser, Urin oder anderen wässrigen Lösungen.

Da diese Produkte in pulverartiger Form vorliegen, ist ihre Einarbeitung in die Endprodukte (z.B. Windeln, Binden usw.) schwierig und erfordert komplizierte und teuere Dosier- und Eintragungsaggregate. Besonders ein feingemahlenes Produkt mit Korngröße unter 100 µm läßt sich sehr schwer verarbeiten. Das pulverförmige Produkt haftet nur schlecht an der Unterlage, so daß in der Regel ein Kleber verwendet werden muß, wodurch wieder ein Teil des Absorptionsvermögens von dem pulverförmigen Produkt verloren geht.

In der FR-A-23 19 434 wird ein Verfahren zum Beschichten eines Bandes aus Papier oder Regeneratcellulose mit einem pulverförmigen, modifizierten, weitgehend wasserunlöslichen Celluloseester beschrieben, wobei die Oberfläche des Bandes befeuchtet (z.B. durch kurzes Eintauchen in Wasser) und anschließend mit dem pulverförmigen Celluloseester bestreut und danach getrocknet wird. Nach einer weiteren Ausführungsform kann der modifizierte Celluloseester in einem organischen Lösungsmittel, gegebenenfalls unter Wasserzusatz, dispergiert werden, worauf dann diese Dispersion auf den Träger (Papier oder Cellulose) aufgebracht wird. Das Absorptionsvermögen eines solchen Materials ist jedoch für viele Zwecke nicht ausreichend. In der FR-A-2 080 724 wird ein Verfahren zur Herstellung eines wasserabsorbierenden Schichtkörpers beschrieben, bei dem unter Druck auf einen faserförmigen Träger ein in Wasser praktisch unlösliches, wassergequollenes Polymergel aufgebracht und dann zwecks Fixierung des Polymeren auf dem Träger auf 100 bis 200° C erhitzt wird. Alternativ kann das Polymergel durch direkte Polymerisation von z. B. Acrylamid in Gegenwart eines Vernetzers und in Gegenwart von Cellulosefasern hergestellt werden, woraus mit dieser Mischung dann der Träger, z.B. ein Papier oder eine Leinwand, beschichtet wird. Auch bei diesem Verfahren ist das Absorptionsvermögen des erhaltenen Absorptionsmaterials für viele Zwecke nicht ausreichend. Weiter ist die Herstellung des Materials verfahrenstechnisch aufwendig, da sich das polymere Absorptionsmittel nur unter vergleichsweise hohem Druck aufbringen läßt und danach anschließend sehr lange getrocknet werden muß. Auch das Absorptionsvermögen dieses Materials ist für viele Zwecke zu niedrig.

Es sind auch Verfahren zur Herstellung von saugfähigen Mischfasern bekannt, nach denen eine polymere hydrophile Komponente, wie z.B. Polyacrylsäure, Carboxymethylcellulose usw., der Spinnlösung zugemischt wird, so daß Fasern mit einer erhöhten Saugfähigkeit entstehen, wie dies z.B. aus DE-OS 2 550 345, DE-OS 2 750 622, DE-OS 2 750 900, DE-OS 2 751 833, DE-OS 2 905 424, DE-OS 3 036 415 und DE-PS 2 634 994 zu entnehmen ist. Die hydrophile Komponente muß in Wasser und in der Spinnlösung vollständig löslich sein. Da das Gewichtsverhältnis der hydrophilen Polymerkomponente zu der Faserkomponente bei diesen Mischfasern immer kleiner als 1 ist, wird dadurch auch das Wasseraufnahmevermögen stark begrenzt. Nach der veröffentlichten Europa-Anmeldung 0023561 wird eine verbesserte Saugfähigkeit von Cellulosefasern dadurch erzielt, daß man sie in einem inerten Medium in teilvernetzte anoxydierte Carboxyalkylcellulose überführt. Die veröffentlichte Europa-Anmeldung 0009322 beschreibt die Herstellung eines absorbierenden Papiers aus Gemischen von Zellstoffasern mit wasserunlöslichen Fasern aus Amin-Formaldehyd-Harz, wobei bestimmte 'Freeness'-(=Mahlgrad)-Verhältnisse eingehalten werden müssen.

Wegen des hohen Wasserquellvermögens der polymeren Absorptionsmittel ist es recht kompliziert, diese Produkte aus wässriger Suspension, wie z.B. bei der Papierherstellung üblich, in das Fertigprodukt einzuarbeiten. Entweder müssen bei der Herstellung des absorbierenden Materials Faserstoffaufschlämmungen mit sehr niedrigem Feststoffgehalt (unter 0,1 %, s.DE-OS 3 037 507) angewendet werden oder die Einarbeitung des Absorptionsmittels in das Fertigprodukt kann (aber nur bei einem carboxylhaltigen Absorptionsmittel) in zwei Phasen erfolgen:

Zunächst wird das Absorptionsmittel in seiner sauren Form gemeinsam mit den Faserkomponenten in Wasser suspendiert, auf einer Papiermaschine die Papierbahn gebildet und getrocknet; die entstandene Papierbahn wird dann in einem weiteren Verfahrensschritt neutralisiert, um das gewünschte Quellvermögen des Fertigprodukts zu erreichen. Das maximale Gewichtsverhältnis der Polymerkomponente zu der Faserkomponente in der Suspension wird mit 65 : 35 angegeben (DE-OS 3 040 964).

Es wäre daher ein Absorptionsmaterial erwünscht, das bei möglichst hohem Absorptionsvermögen auf möglichst einfache Weise herstellbar ist. Besonders erwünscht wäre ein Absorptionsmaterial, bei dem das

2

Absorptionsmittel möglichst fest auf oder in dem Träger, und zwar möglichst ohne zusätzliche Klebstoffe, verankert bzw. fixiert ist. Ganz besonders vorteilhaft wäre ein Absorptionsmaterial, das die Form eines Flächengebildes, z.B. eines Blattes oder Vlieses oder einer Gewebebahn aufweist, wobei das Absorptionsmittel in oder auf dem flächenförmigen Träger fixiert ist. Ein solches Absorptionsmaterial sollte einen möglichst hohen Gehalt an Absorptionsmittel enthalten.

Ausgehend von bekannten Absorptionsmaterialien, die aus einem polymeren Absorptionsmittel und einem Träger bestehen, ist daher Aufgabe der Erfindung die Verbesserung eines solchen Absorptiosmaterials unter Vermeidung von dessen Nachteilen, insbesondere also die Schaffung eines Absorptionsmaterials, bei dem das Absorptionsmittel ohne zusätzliche Klebstoffe, fest in oder auf dem Träger fixiert ist.

Diese Aufgabe wird durch ein Absorptionsmaterial gelöst, das aus einem auf oder in einem Trägermaterial dauerhaft fixierten Absorptionsmittel besteht, wobei das erfindungsgemäße Absorptionsmaterial durch Behandeln des Trägermaterials mit dem mittels eines wasserhaltigen organischen Lösungsmittels wenigstens teilweise gequollenen Absorptionsmittel und Trocknen erhalten worden ist.

Als Trägermaterial sind sowohl Flächengebilde als auch diskrete Partikel geeignet, wobei jedoch für die Zwecke der Erfindung flächenförmige Träger, wie Blätter, Bahnen oder Platten bevorzugt werden. Geeignet sind z.B. Bahnen aus Textilgewebe, Vliese, Papierblätter oder -bahnen, aber auch Glas-, Keramik- und Metallplatten. Für die Zwecke der Erfindung sind besonders bevorzugt Bahnen bzw. Blätter aus Textilgewebe oder Papier, sowie Vliese. An nicht-flächenförmigen Trägermaterialien sind besonders künstliche oder natürliche Fasern, wie Cellulose oder Kunststoffasern geeignet, die zu Flächengebilden verarbeitet werden können. Daneben sind aber auch andere partikelförmige Trägermaterialien, wie z.B. Holzmehl (Sägespäne), geeignet.

Als geeignete Absorptionsmittel, die bevorzugt pulverförmig sind, kommen sowohl die wasserquellbaren Polymeren auf der Basis von Polysacchariden wie Cellulose, Cellulosederivate wie Carboxymethylcellulose, Alkyl- oder Hydroxyalkylcellulose, Stärke und Stärkederivate und Pflanzengummi (z.B. Xanthangummi), Alginsäure und ihre Salze als auch die Polymeren oder Copolymeren auf der Basis von (Meth-)Acrylsäure oder (Meth-)Acrylsäurederivaten infrage, wobei es sich hierbei in erster Linie um die Homo- oder Copolymeren der Acryl-, Methacryl-, Acrylamidomethylpropansulfonsäure, der Salze dieser Säuren, des Acryl- oder Methacrylamids untereinander oder mit Vinylpyrrolidon und/oder Vinylacetat handelt. Die vorstehenden Polymeren können durch einen mindestens bifunktionellen Vernetzer vernetzt sein, damit sie in Wasser nur quellbar, aber nicht löslich sind. Alle diese Polymeren werden nach bekannten Verfahren hergestellt.

Das erfindungsgemäße Absorptionsmaterial besteht aus Faserstoff und Absorptionsmittel in einem Gewichtsverhältnis 10 bis 99,9, vorzugsweise 20 bis 75 Gew.-% des Faserstoffs und 0,1 bis 90, vorzugsweise 25 bis 80 Gew.-% des Absorptionsmittels. Das erfindungsgemäße Absorptionsmaterial kann danach nahezu vollständig aus Absorptionsmittel bestehen und weist dennoch den Vorteil eines Flächengebildes auf.

Das erfindungsgemäße Absorptionsmaterial kann daneben noch Riechstoffe, Bindemittel oder sonstige Hilfsstoffe, wie z.B. Desinfektionsmittel enthalten, sofern sie die Absorptionseigenschaften des Absorptionsmaterials nicht negativ beeinflussen.

Das erfindungsgemäße Absorptionsmaterial wird durch Behandeln des Trägermaterials mit dem wenigstens teilweise gequollenen Absorptionsmittel und nachfolgendem Trocknen hergestellt. Die teilweise Quellung erfolgt mit wenigstens einem wasserhaltigen organischen Lösungsmittel. Anschließend wird getrocknet.

Als organische Lösungsmittel können verschiedene mit Wasser mischbare Lösungsmittel verwendet werden, vorzugsweise Methanol, Ethanol, Isopropanol, Aceton, Tetrahydrofuran, Dioxan, Glycerin, Ethylenglykol, die 0,1 bis 60 % Wasser enthalten können. Nach der einen Herstellungsweise arbeitet man das wenigstens teilweise gequollene Absorptionsmittel in einen teilchenförmigen Träger, der bevorzugt ein Faserstoff wie Cellulose oder eine Textilfaser ist, ein, verformt die Mischung zu einem Flächengebilde und trocknet. Hierbei erhält man gleich ein flächenförmiges Absorptionsmaterial. Bei dieser Herstellungsweise wird zweckmäßig das Absorptionsmittel dem Faserstoff in Gegenwart eines wasserhaltigen organischen Lösungsmittels zugemischt. Dabei wird der faserförmige Träger in einem organischen Lösungsmittel, das bis zu 60 % Wasser enthalten kann (für praktische Zwecke genügt ein Wassergehalt von 10 - 20 %) aufgeschlämmt und mit dem pulverförmigen Absorptionsmittel, das zur besseren Mischbarkeit vorzugsweise ebenfalls im organischen, gegebenenfalls wasserhaltigen Lösungsmittel suspendiert ist, vermischt. Aus der Aufschlämmung wird eine Faserbahn hergestellt, wozu beispielsweise die Aufschlämmung auf ein Sieb oder eine Nutsche aufgebracht, das überschüssige Lösungsmittel gegegenenfalls mit Hilfe von Unterdruck entfernt und das entstandene Blatt oder Vlies getrocknet wird. Die Trocknung kann bei Normaltemperatur, vorzugsweise bei erhöhten Temperaturen, durchgeführt werden.

Überraschenderweise wirken die teilweise aufgequollenen Teilchen des Absorptionsmittels gleichzeitig als Bindemittel und Trockenverfestiger für den Faserstoff des Trägermaterials. Es wird also gleichzeitig ein flächenförmiges Absorptionsmaterial mit einer einstellbaren, im Bedarfsfall außerordentlich hohen Menge Absorptionsmittel ohne die Verwendung eines zusätzlichen Bindemittels und Trockenverfestigers für das Flächengebilde, wie beispielsweise sonst bei der Papierherstellung üblich, möglich. Man erhält so je nach dem Quellungsgrad der Absorptionsmittelteilchen, der ebenfalls durch Lösungsmittel und/ oder Wassergehalt gesteuert werden kann, Papierblätter oder Vliese mit verschiedener Trockenfestigkeit, in denen das Absorptionsmittel integraler Bestandteil des Flächengebildes ist und somit fest und dauerhaft ohne zusätzliche Hilfsstoffe fixiert ist.

Auch andere partikelförmige Träger, wie Sägespäne, können auf diese Weise zu einem flächenförmigen Absorptionsmaterial verarbeitet werden. Solche Produkte können z.B. zur Bodenverbesserung bzw.-befestigung verwendet werden.

Überraschenderweise erreicht man durch die Behandlung eines Trägers der, wie Watte, an sich nur eine geringe Zugfestigkeit hat, durch die Behandlung mit dem Absorptionsmittel eine sehr starke Verbesserung der Festigkeitseigenschaften.

Es muß ferner überraschen, daß besonders vorteilhafte Ergebnisse mit pulverförmigen Absorptionsmitteln einer Korngröße kleiner als 200, insbesondere kleiner als 100 µm erzielt werden.

Solche Korngrößen lassen sich im pulverförmigen Zustand nur schwer verarbeiten.

Nach einer weiteren Herstellungsweise wird das erfindungsgemäße Absorptionsmaterial erhalten, indem man das wenigstens teilweise in einem wasserhaltigen organische Lösungsmittel gequollenen Absorptionsmittel auf die Oberfläche eines Trägers, vorzugsweise eines flächenförmigen Trägers, aufbringt. Der Träger kann hierbei trocken oder ebenfalls angefeuchtet sein. Das Auftragen des gequollenen Absorptionsmittels kann durch Aufsprühen oder Aufstreichen erfolgen, worauf getrocknet wird. Das Absorptionsmittel wird vorteilhaft in Form einer Suspension im wäßrig organischen Lösungsmittel aufgebracht.

Bei beiden Herstellungsvarianten erfolgt die Fixierung des Absorptionsmittels in oder auf dem Trägermaterial durch Trocknung und/oder Druckanwendung, wobei im Regelfall zusätzliche Bindemittel zwecks Fixierung des Absorptionsmittels auf dem Träger überflüssig sind.

Das erfindungsgemäße Absorptionsmaterial ist aufgrund seiner Zusammensetzung zur Aufnahme und/oder Zurückhaltung von Wasser, Wasserdampf, wässrigen Lösungen und Körperflüssigkeiten, wie z.B. Urin oder Blut geeignet, besonders für den Einsatz in absorbierenden Wegwerferzeugnissen wie Babywindeln, Damenbinden, Tampons oder in absorbierenden Erzeugnissen für chirurgische und medizinische Verwendung.

Weiterhin kann das mit Wasser oder wässrigen Lösungen aufgequollene erfindungsgemäße Absorptionsmaterial zur gesteuerten Abgabe von Wasser und/oder den in Wasser gelösten Komponenten an andere Körper, wie z.B. an Pflanzen sowie als Nährboden für verschiedene Kulturen, beim gezielten Dosieren der Medikamente und ebenfalls im technischen Bereich (Akkumulatorflüssigkeiten, Filteranlagen usw.) verwendet werden.

Die Erfindung wird durch die folgenden Beispiele veranschaulicht.

**Beispiel 1:**

In einem Gefäß wurden 3 g Cellulose in 300 ml 96 %-igem Äthanol suspendiert und mit einem schnellaufenden Schlagrührwerk aufgeschlagen. Danach wurden 3 g Absorptionsmittel (vernetzte Polyacrylsäure) in 50 ml Äthanol suspendiert und mit den aufgeschlagenen Cellulosefasern vermischt. Das Gemisch wurde dann auf eine Nutsche (Ø 20 cm) aufgetragen, das Lösungsmittel entfernt und das entstandene Blatt bei 85°C getrocknet.

Das Blatt hatte folgende Zusammensetzung:

| | |
|---|---|
| Cellulosefaser | 95 g/m$^2$ |
| Absorptionsmittel | 95 g/m$^2$ |
| Gesamtflächengewicht | 190 g/m$^2$ |

Das Saugvermögen des erfindungsgemäßen Produkts wurde auf einem 16 cm$^2$ großen Blatt mit Wasser und Modellurinlösung (2,0 % Harnstoff, 0,9 % NaCl, 0,1 % MgSO$_4$ und 0,06 % CaCl$_2$ aufgelöst in destilliertem Wasser) geprüft.

Das Absorptionsvermögen dieses Blattes betrug
31,1 ml Modellurinlösung/g
198 ml Wasser/g

**Beispiel 2:**

Gemäß Beispiel 1 wurden in einem Gefäß 3g Cellulose in 300 ml 96 %-igem Äthanol suspendiert und mit einem schnellaufenden Schlagrührwerk aufgeschlagen. Danach wurden 9g Absorptionsmittel (vernetzte Polyacrylsäure) in 50 ml 96 %-igem Äthanol suspendiert und mit den aufgeschlagenen Cellulosefasern vermischt. Das Gemisch wurde dann auf eine Nutsche (Ø 20 cm) aufgetragen, das Lösungsmittel entfernt und das entstandene Blatt bei 85°C getrocknet.

Das Blatt hatte folgende Zusammensetzung:

| Gellulosefaser | 95 g/m² |
| Absorptionsmittel | 285 g/m² |
| Gesamtflächengewicht | 380 g/m² |

Das Absorptionsvermögen - geprüft wie im Beispiel 1 - dieses Blattes betrug
35,5 ml Modellurinlösung/g
202 ml Wasser/g

**Beispiel 3:**

Gemäß Beispiel 2 wurden 3 g Cellulose in verschiedenen organischen Lösungsmitteln sowie in wasserhaltigen organischen Lösungsmitteln aufgeschlagen, mit 9 g polymerem Absorptionsmittel (vernetzte Polyacrylsäure) vermischt, in gleicher Weise verarbeitet und geprüft wie im Beispiel 2. Die mechanische Trockenfestigkeit des Blattes wurde mit den Berstfestigkeitsprüfer ermittelt, wobei das Blatt auf einer Papierunterlage mit Berstfestigkeit von 15,0 N/cm² durch Anpressen fixiert wurde.

| Lösungsmittel | Absorptionsvermögen Modellurinlösung | (ml/g) Wasser | Berstfestig-keit N/cm² |
|---|---|---|---|
| Toluol | 23,8 | 150 | 15,0* |
| Tetrahydrofuran | 26,3 | 171 | 15,5* |
| Tetrahydrofuran/Wasser 90/10 | 35,6 | 188 | 18,0 |
| Dioxan/Wasser 98/2 | 35,9 | 175 | 16,0 |
| Aceton | 29,2 | 151 | 15,1* |
| Aceton/Wasser 90/10 | 40,9 | 152 | 18,8 |
| Methanol/Wasser 98/2 | 32,7 | 185 | 22,5 |
| Methanol/Wasser 95/5 | 32,5 | 198 | 23,5 |
| Methanol/Wasser 90/10 | 26,7 | 195 | 25,3 |
| Methanol/Wasser 80/20 | 36,3 | 190 | 24,0 |
| Äthanol/Wasser 95/5 | 37,5 | 210 | 23,7 |
| Äthanol/Wasser 90/10 | 38,3 | 201 | 25,3 |

*Keine Bindung zwischen dem Faserstoff und dem Absorptionsmittel ist entstanden.

**Beispiel 4:**

Gemäß Beispiel 2 wurden 3 g gemahlene Cellulose mit verschiedenen Mengen an polymerem Absorptionsmittel (vernetzte Polyacrylsäure-Natriumsalz) im Methanol/Wasser-Gemisch 90/10 vermischt, in gleicher Weise verarbeitet und geprüft wie in Beispiel 3:

| Zusammensetzung | | | Absorptionsvermögen (ml/g) | |
|---|---|---|---|---|
| Cellulose-faser g/m² | Absorptions-mittel g/m² | Gesamtflächen-gewicht g/m² | Modellurin-lösung | Wasser |
| 95 | 32 | 127 | 31,4 | - |
| 95 | 95 | 190 | 35,7 | 195 |
| 95 | 380 | 475 | 40,8 | - |
| 95 | 570 | 665 | 37,2 | - |
| 95 | 860 | 955 | 38,4 | 120 |

**Beispiel 5:**

Gemäß Beispiel 2 wurden 3 g gemahlene Cellulose mit verschiedenen Absorptionsmitteln (jeweils 9,0 g) im Methanol/ Wasser-Gemisch 90/10 vermischt und in gleicher Weise verarbeitet wie in Beispiel 3.

| Asorptionsmittel | Absorptionsvermögen (ml/g) | | Berstfestigkeit N/cm$^2$ |
|---|---|---|---|
| | Modellurinlösung | Wasser | |
| Polyacrylamid Mol.gew. 5.10$^6$ g/ml | 5,1 | 4,8 | 20,3 |
| Acrylamid/Acrylsäure Copolymerisat 65/35 Mol.gew. 6.10$^6$ g/ml | 5,5 | - | 23,5 |
| Carboxymethyl-cellulose | 7,7 | 8,8 | - |
| Alginat-Calcium/ Natriumsalz | 10,5 | 10,5 | - |
| Methylhydroxyethyl-cellulose | 5,4 | 5,1 | 25,0 |
| vernetztes Stärke-Acrylsäure-Copoly-merisat | 15,5 | 45,1 | - |

**Beispiel 6:**

Gemäß Beispiel 2 wurden 3 g Zellwolle-Stapelfaser 1,7/6 dtex/mm mit 8 g polymerem Absorptionsmittel (vernetzte Polyacrylsäure) in Methanol/Wasser-Gemisch 90/10 vermischt und in gleicher Weise verarbeitet wie in Beispiel 2. Neben dem Wasser- und Modellurinabsorptionsvermögen wurde noch das Absorptionsvermögen mit Humanblut unter Belastung (32 g/cm$^2$) geprüft.
Das Absorptionsvermögen dieses Materials betrug
152 ml Wasser/g
36,6 ml Modellurinlösung/g
3,9 ml Blut/g

**Beispiel 7:**

Gemäß Beispiel 2 wurden 1,5 g Zellwolle-Stapelfaser 1,7/6 dtex/mm mit 1,5 g Cellulose und 9 g polymerem Absorptionsmittel (vernetzte Polyacrylsäure) in Methanol/ Wasser-Gemisch 90/10 vermischt und in gleicher Weise verarbeitet wie in Beispiel 2. Neben dem Wasser- und Modellurinabsorptionsvermögen wurde noch das Absorptionsvermögen mit Humanblut unter Belastung (32 g/cm$^2$ ) geprüft. Das Absorptionsvermögen dieses Materials betrug
120 ml Wasser/g
22,5 ml Modellurinlösung/g
3,8 ml Blut/g

**Beispiel 8:**

Auf ein Vlies, hergestellt aus Zellwolle-Stapelfaser 1,7/34 dtex/mm wurde ein vernetztes Polyacrylsäurenatriumsalz als Suspension in Methanol/Wasser-Gemisch 90/10 aufgesprüht und durch Trocknung bei 85°C auf dem Vlies fixiert. Das beschichtete Vlies hatte folgende Zusammensetzung:

| | |
|---|---|
| Cellulosefaser | 78 g/m$^2$ |
| Absorptionsmittel | 23 g/m$^2$ |

Das Absorptionsvermögen dieses Materials betrug
65 ml Wasser/g
21,6 ml Modellurinlösung/g
3,5 ml Blut/g

**Beispiel 9:**

Auf ein Vlies, hergestellt aus Polypropylen-Stapelfaser 1,7/45 dtex/mm wurde vernetztes Polyacrylsäurenatriumsalz als Suspension in Methanol/Wasser-Gemisch 90/10 aufgesprüht und durch Trocknung bei 85°C auf dem Vlies fixiert. Das beschichtete Vlies hatte folgende Zusammensetzung:

Polypropylenfaser        70 g/m²
Absorptionsmittel        21 g/m²

Das Absorptionsvermögen dieses Materials betrug
58 ml Wasser/g
20,5 ml Modellurinlösung/g

**Beispiel 10:**

Eine Paste, vorbereitet aus 50 g vernetztem Acrylsäure/ Acrylamidomethylpropansäure-Copolymerisat 90/10 in 100 g Methanol/Glycerin/Wasser-Gemisch 50/40/10, wurde auf ein Vlies, das aus Polyester-Stapelfaser 1,7/34 dtex/mm hergestellt wurde, in dünner Schicht im Streichverfahren aufgetragen und das Absorptionsmittel durch Trocknung bei 95°C auf dem Vlies fixiert. Das beschichtete Vlies enthielt 55 g Absortionsmittel/m² und sein Absorptionsvermögen betrug
112 ml Wasser/g oder
36 ml Modellurinlösung/g.

**Beispiel 11:**

Auf ein Filterpapier (Ø 18 cm, 85 g/m²) wurde ein vernetztes Acrylsäurenatriumsalz/Acrylamid-Copolymerisat 90/10 als Suspension in Äthanol/Wasser-Gemisch 95/5 aufgetragen und bei 90°C getrocknet. Das Material enthielt 60 g des Absorptionsmittels pro m² und sein Absorptionsvermögen betrug
19,2 ml Modellurinlösung/g
55 ml Wasser/g.

**Beispiel 12:**

Auf 100 cm² große Glasplatten wurde 0,75 g eines vernetzten Polyacrylsäurenatriumsalzes als Suspension in Äthanol/Wasser-Gemisch 90/10 aufgesprüht und durch Trocknung bei Normaltemperatur auf den Platten fixiert. Die getrocknete Schicht des Absorptionsmittels bindet 217 g Wasser oder 29,5 ml Modellurinlösung.

**Beispiel 13:**

In einem Gefäß wurden 10 g Holzmehl mit einer Suspension von 10 g Absorptionsmittel (vernetzte Polyacrylsäure) in 20 g Methanol/Wasser-Gemisch bei Normaltemperatur 10 min behandelt und danach bei 80°C getrocknet. Das Absorptionsvermögen dieses Absorptionsmaterials betrug
65 ml Wasser/g
18,5 ml Modellurinlösung/g
3,1 ml Blut/g

**Beispiel 14:**

Ein Papierblatt (80 g/m²) wurde mit 80 g/m² Wasser/Methanol-Gemisch 60/40 befeuchtet und mit feinpulverisiertem vernetzten Polyacrylsäure-Natriumsalz (Körnung < als 100 μm) bestreut und danach bei 100°C getrocknet. Das Material enthielt 60 g des Absorptionsmittels pro m². Das Absorptionsvermögen dieses Blattes betrug
10.5 ml Modellurinlösung/g
12,5 ml Wasser/g

**Beispiel 15:**

In einem Gefäß wurden 200 g Torf mit einer Suspension von 200 g vernetzter Polyacrylsäure in 3600 g Äthanol/Wasser-Gemisch 90/10 bei Normaltemperatur 15 min behandelt und danach bei 90°C getrocknet. Es entstand ein Material mit abriebfestgebundenem Absorptionsmittel, dessen Absorptionsvermögen betrug:
122 ml Wasser/g
44 ml 0,1 %-ige NaCl-Lösung
24 ml 1,0 %-ige NaCl-Lösung
27 ml Modellurinlösung

**Beispiel 16:**

Herstellung eines vernetzten synthetischen Polymerisates:
In einem Polymerisationsgefäß wurden 328 g Acrylsäure, 2,6 g N,N'-Methylenbisacrylamid in 980 g Wasser gelöst und mit 127,5 g Natriumhydrogencarbonat auf pH = 4,0 eingestellt. Bei normaler Temperatur wurden die Komponenten des Katalysatorsystems (0,36 g Azobisamidinpropandihydrochlorid, 0,73 g Kaliumpersulfat, 1,34 g Natriumpyrosulfit und 0,06 g Eisen(II)-gluconat), gelöst in 120 ml Wasser, zugegeben, wobei adiabatische Polymerisation erfolgt. Das entstandene Polymergel wurde zerkleinert, getrocknet und gemahlen.

**Beispiel 17:**

Herstellung eines vernetzten synthetischen Copolymerisates:
In einem Polymerisationsgefäß wurden 320 g Acrylsäure, 56 g Vinylpyrrolidon und 3,75 g N,N'-Methylenbisacrylamid in 862 g Wasser gelöst und mit 100 g Natriumhydrogencarbonat auf pH = 4,4 neutralisiert. Bei normaler Temperatur wurden die einzelnen Komponenten des Katalysatorsystems (0,6 g Azobisamidinpropandihydrochlorid, 1,2 g Natriumpyrosulfit und 0,6 g Kaliumpersulfat), gelöst in 150 g Wasser, zudosiert. Die Polymerisation erfolgt praktisch adiabatisch. Das entstandene Polymergel wurde zerkleinert, getrocknet und gemahlen.

**Patentansprüche**

1. Verfahren zur Herstellung eines Absorptionsmaterials zur Aufnahme von Wasser, wäßrigen Lösungen und wäßrigen Körperflüssigkeiten, das aus einem auf oder in einem Träger fixierten Absorptionsmittel auf Basis eines Polymeren oder Copolymeren von (Meth-)Acrylsäure oder eines (Meth-)Acrylsäurederivats besteht, durch Behandeln des Trägermaterials mit dem Absorptionsmittel in Gegenwart von Wasser und anschließender Trocknung, dadurch gekennzeichnet, dass man das Absorptionsmittel in einem wasserhaltigen organischen Lösungsmittel wenigstens teilweise quillt, in das partikelförmige Trägermaterial einarbeitet und die so erhaltene Mischung vor dem Trocknen verformt.

2. Verfahren nach Anspruch 1, dadurch gekennzeichnet, daß man die teilweise Quellung des Absorptionsmittels in Gegenwart des Trägermaterials vornimmt, in dem das Absorptionsmittel dem teilchenförmigen Träger in Gegenwart des wasserhaltigen organischen Lösungsmittels zugemischt wird.

3. Verfahren nach einem der Ansprüche 1 bis 2, dadurch gekennzeichnet, daß als teilchenförmiges Trägermaterial eine natürliche oder synthetische Faser oder Holzmehl oder Sand verwendet wird.

4. Verfahren zur Herstellung eines Absorptionsmaterials zur Aufnahme von Wasser, wäßrigen Lösungen und wäßrigen Körperflüssigkeiten, das aus einem auf oder in einem Träger fixierten Absorptionsmittel auf Basis eines Polymeren oder Copolymeren von (Meth-)Acrylsäure oder eines (Meth-)Acrylsäurederivates besteht, durch Behandeln des Trägermaterials mit dem Absorptionsmittel in Gegenwart von Wasser und anschließender Trocknung, dadurch gekennzeichnet, daß man das Absorptionsmittel auf ein geformtes Trägermaterial aufbringt, vor oder nach dem Aufbringen in einem wasserhaltigen organischen Lösungsmittel wenigstens teilweise quillt und anschließend trocknet.

5. Verfahren nach Anspruch 4, dadurch gekennzeichnet, daß man das Absorptionsmittel in Form einer Suspension im wasserhaltigen organischen Lösungsmittel auf das geformte Trägermaterial aufbringt.

6. Verfahren nach einem der Ansprüche 4 bis 5, dadurch gekennzeichnet, daß man als Trägermaterial ein Flächengebilde verwendet.

7. Verfahren nach einem der Ansprüche 4 bis 6, dadurch gekennzeichnet, daß als Trägermaterial ein Textilgewebe oder Vlies verwendet wird.

8. Verfahren nach einem der Ansprüche 1 bis 7, dadurch gekennzeichnet, daß Trägermaterial und Absorptionsmittel in Mengen von 10 bis 99,9 % und 0,1 bis 90 Gew.-% angewendet werden.

9. Verfahren nach Ansprüchen 1 bis 8, dadurch gekennzeichnet, daß man als organisches, wasserhaltiges Lösungsmittel Alkohole, Aceton oder Tetrahydrofuran mit einem Wassergehalt von 0,1 bis 60 Vol.-% verwendet.

10. Verfahren nach Ansprüchen 1 bis 9, dadurch gekennzeichnet, daß als Absorptionsmittel ein Homo- oder Copolymeres der Acrylsäure, Methacrylsäure, Acrylamidomethylpropansulfonsäure, der Salze dieser Säuren, des Acryl- oder Methacrylamids untereinander oder mit Vinylpyrroliden und/oder Vinylacetat verwendet wird.

11. Verwendung des Absorptionsmaterials, hergestellt nach Ansprüchen 1 bis 10, zur Aufnahme und/oder Zurückhaltung von Wasser, Wasserdampf und/oder wäßrigen Lösungen in absorbierenden Wegwerferzeugnissen für hygienische, chirurgische und andere medizinische Zwecke.

12. Verwendung des Absorptionsmaterials, hergestellt nach Ansprüchen 1 bis 10, zur Aufnahme und/oder Zurückhaltung von Wasser oder wäßrigen Lösungen und zur nachfolgenden gesteuerten Abgabe von Wasser und/ oder der in Wasser gelösten Komponenten an Pflanzen, als Nährböden für verschiedene Kulturen, beim Dosieren von Medikamenten, für Akkumulatorflüssigkeiten oder Filteranlagen.

13. Verwendung des Absorptionsmaterials, hergestellt nach Ansprüchen 1 bis 10, als Filtereinlage bei Rauchartikeln zur Aufnahme von in Rauch enthaltenen Destillationsprodukten.

## Claims

1. A process for the production of an absorption material for the absorption of water, aqueous solutions and aqueous body fluids, comprising an absorbent being fixed on or in a support, said absorbent being based on a polymer or copolymer of (meth-)acrylic acid or a (meth-)acrylic acid derivative, by treating the support material with the absorption material in the presence of water and subsequent drying, characterized in that the absorption material is swollen at least partially in a water-containing organic solvent, worked in the particle-shaped support material and the mixture received deformed before drying.

2. A process according to claim 1, characterized in that the partial swelling of the absorption material is carried out in the presence of the support material by adding the absorption material to the particle- shaped support in the presence of the water-containing organic solvent.

3. A process according to claims 1 to 2, characterized in that a natural or synthetic fabric or wood flour or sand is used as support material.

4. A process for the production of an absorption material for the absorption of water, aqueous solutions and aqueous body fluids, comprising an absorbent being fixed on or in a support, said absorbent being based on a polymer or copolymer of (meth-)acrylic acid or a (meth-)acrylic acid derivative, by treating the support material with the absorption material in the presence of water and following drying, characterized in that the absorption material is applied on a shaped support material, swollen at least partially before or after it was applied to the water-containing organic solvent, and dryed subsequently.

5. A process according to claim 4, characterized in that the absorbent is applied in the form of a suspension in the water-containing solvent on the shaped support material.

6. A process according to claims 4 to 5, characterized in that a flat material is used as support material.

7. A process according to claims 4 to 6, characterized in that a textile fabric or a fleece is used as support material.

8. A process according to claims 1 to 7, characterized in that support material and absorption material are used in amounts of 10 to 99.9 % and 0.1 to 90 %-wt.

9. A process according to claims 1 to 8, characterized in that alcohols, acetone or tetrahydrofurane having a water content of 0.1 to 60 % by volume are used as organic water-containing solvents.

10. A process according to claims 1 to 9, characterized in that a homo- or copolymer of acrylic acid, methacrylic acid, acrylamidomethylpropanesulphonic acid, the salts of said acids, acrylic amide or methacrylic amide with one another or with vinylpyrrolidone and/or vinyl acetate are used as absorption material.

11. The use of the absorption material manufactured according to claims 1 to 10 for the absorption and/or retention of water, water vapour and/or aqueous solutions in absorbing disposable products for hygienical, surgical and other medical purposes.

12. The use of the absorption material manufactured according to claims 1 to 10 for the absorption and/or retention of water or aqueous solutions and subsequent controlled release of water and/or components dissolved in water to plants as nutrient mediums for various cultures, for the dosage of medicals, for accumulator liquids and filter systems.

13. The use of the absorption material manufactured according to claims 1 to 10 as filters in smoking articles for the absorption of distillation products contained in the smoke.

## Revendications

1. Procédé de préparation d'un matériau absorbant pour l'absorption d'eau, de solutions aqueuses et de liquides corporels aqueux, constitué d'un agent absorbant à base d'un polymère ou d'un copolymère d'acide (méth) acrylique ou d'un dérivé d'acide (méth)acrylique, fixé sur ou dans un support, par un traitement du

**077 510**

matériau support en présence d'eau avec l'agent absorbant, suivi d'un séchage, caractérisé en ce que l'agent absorbant est gonflé au moins partiellement dans un solvant organique aqueux, puis mélangé au matériau support en particules, le mélange obtenu étant ensuite façonné, puis séché.

2. Procédé suivant la revendication 1, caractérisé en ce que le gonflement partiel de l'agent absorbant est réalisé en présence du matériau support en mélangeant l'agent absorbant et le support en particules en présence du solvant organique aqueux.

3. Procédé suivant l'une des revendications 1 et 2, caractérisé en ce que le matériau support en particules est choisi parmi les fibres naturelles et synthétiques, la farine de bois et le sable.

4. Procédé de préparation d'un matériau absorbant pour l'absorption d'eau, de solutions aqueuses et de liquides corporels aqueux, constitué d'un agent absorbant à base d'un polymère ou d'un copolymère d'acide (méth) acrylique ou d'un dérivé d'acide (méth) acrylique, fixé sur ou dans un support, par un traitement du matériau support en présence d'eau avec l'agent absorbant, suivi d'un séchage, caractérisé en ce que l'agent absorbant est appliqué sur un matériau support, étant gonflé au moins partiellement, avant ou après son application, à l'aide d'un solvant organique aqueux, puis séché.

5. Procédé suivant la revendication 4, caractérisé en ce que l'agent absorbant est déposé sous forme d'une suspension dans un solvant organique aqueux sur le matériau support façonné.

6. Procédé suivant l'une des revendications 4 et 5, caractérisé en ce que le matériau support est une structure plane.

7. Procédé suivant l'une des revendications 4 à 6, caractérisé en ce que le matériau support est constitué d'un tissu textile ou d'un mat (de fibres).

8. Procédé suivant l'une des revendications 1 à 7, caractérisé en ce que le matériau support et l'agent absorbant sont utilisés en des proportions de 10 à 99,9 % et de 0,1 à 90 % en poids.

9. Procédé suivant les revendications 1 à 8, caractérisé en ce que l'on emploie comme solvant organique aqueux un alcool, de l'acétone ou le tétrahydrofuranne avec une proportion d'eau comprise entre 0,1 et 60 % en volume.

10. Procédé suivant les revendications 1 à 9, caractérisé en ce que l'agent absorbant est un homo- ou un copolymère de l'acide acrylique, de l'acide méthacrylique ou d'acide acrylamido-méthyl-propane-sulfonique ou de sels de ces acides, de l'amide acrylique et de l'amide méthacrylique entre eux ou avec la vinyl-pyrrolidone et(ou) l'acétate de vinyle.

11. Utilisation d'un matériau absorbant, préparé (par le procédé) selon les revendications 1 à 10, pour l'absorption et (ou) la rétention d'eau, de vapeur d'eau et(ou) de solutions aqueuses dans des produits absorbants à usage hygiénique, chirurgical ou généralement médical, à jeter après l'emploi.

12. Utilisation d'un matériau absorbant, préparé (par le procédé) selon les revendications 1 à 10, pour l'absorption et(ou) la rétention d'eau ou de solutions aqueuses et cession réglable subséquente de cette eau et(ou) des substances dissoutes dans celle-ci à des plantes, comme milieu nutritif pour diverses cultures, pour le dosage de médicaments, pour liquides d'accumulateurs (batteries) et dans des installations de filtration.

13. Utilisation d'un matériau absorbant, préparé (par le procédé) selon les revendications 1 à 10, comme matériau filtrant dans des produits à fumer pour l'absorption des produits de distillation contenus dans la fumée.